# EUROPÄISCHE PATENTANMELDUNG

(11) **EP 1 798 545 A1**
(43) Veröffentlichungstag der Anmeldung: **20.06.2007**
(21) Anmeldenummer: 06024495.1
(22) Anmeldetag: 27.11.2006
(51) Int. Cl.: G01N 27/411, G01N 33/20

(54) **Messsonde**

(30) Priorität: 15.12.2005 DE 102005060493
(71) Anmelder: Heraeus Electro-Nite International N.V., 3530 Houthalen (BE)
(72) Erfinder: Neyens, Guido Jacobus, 3680 Opoeteren (BE)
(74) Vertreter: Kühn, Hans-Christian

(57) **Zusammenfassung**

Die Erfindung betrifft eine Messsonde zur Messung in Metallschmelzen mit einem an einem Eintauchende eines Trägerrohres angeordneten Messkopf, an dem mindestens ein Sensor zur Bestimmung einer Komponente der Metallschmelze und ein Badkontakt (7) angeordnet sind, wobei der Badkontakt einen, in Eintauchrichtung gesehen, vorderen Badkontakt-Bereich aufweist mit jeweils zwei senkrecht zur Eintauchrichtung gegenüberliegend angeordneten Oberflächenbereichen, und wobei an den gegenüberliegend angeordneten Oberflächenbereichen des vorderen Badkontakt-Bereichs ein in Eisen- oder Stahlschmelze beständiges Material (9) angeordnet ist und wobei an einem hinteren Badkontakt-Bereich lediglich an höchstens einem Oberflächenbereich ein in Eisen- oder Stahlschmelze beständiges Material angeordnet ist, wobei der gegenüberliegend angeordnete Oberflächenbereich des hinteren Badkontakt-Bereiches frei von diesem Material ist.

## Beschreibung

Die Erfindung betrifft eine Messsonde zur Messung in Metall- oder Schlackeschmelzen mit einem an einem Eintauchende eines Trägerrohres angeordneten Messkopf, an dem mindestens ein Sensor zur Bestimmung einer Komponente der Metallschmelze und ein Badkontakt angeordnet sind, wobei der Badkontakt einen, in Eintauchrichtung gesehen, vorderen Badkontakt-Bereich aufweist mit jeweils zwei senkrecht zur Eintauchrichtung gegenüberliegend angeordneten Oberflächenbereichen.

Derartige Messsonden sind vielseitig bekannt. So sind in DE 3541806 C1 oder in DE 83 17 643 U1 Messsonden bekannt, an deren Eintauchseite ein Badkontakt angeordnet ist. Der Badkontakt ist einer elektrochemischen Messzelle zugeordnet. Er ist als Metallstab ausgebildet. Aus DE 29 54 228 C2 und DE 79 25 016 U1 bekannt, die rohrförmig ausgebildet sind und die jeweils einen Schenkel eines Sensors umfassen. Der Badkontakt kann neben dem Zusammenwirken mit einem elektrochemischen Element auch zur Messung der Badhöhe einer Metallschmelze verwendet werden, beispielsweise in der Art, dass beim Eintauchen des Badkontaktes in die Metallschmelze ein Stromkreis geschlossen und über die Position des Badkontaktes die Höhe der Metallschmelze bestimmt wird. Der Badkontakt ist in der Regel aus Stahl und wird innerhalb der Metallschmelze nach kurzer Zeit zerstört.

Aufgabe der vorliegenden Erfindung ist es, die bekannten Messsonden und insbesondere die dabei verwendeten Badkontaktanordnungen zu verbessem und damit die Zuverlässigkeit der Messsonden zu erhöhen.

Die Aufgabe wird erfindungsgemäß durch den Gegenstand des Anspruchs 1 gelöst. Vorteilhafte Ausgestaltungen ergeben sich aus den Unteransprüchen.

Dadurch, dass an den gegenüberliegenden Oberflächenbereichen des vorderen Badkontakt-Bereiches ein in Eisen- oder Stahlschmelze beständiges Material angeordnet ist und dass an einem hinteren Badkontakt-Bereich lediglich an einem Oberflächenbereich ein in Eisen- oder Stahlschmelze beständiges Material angeordnet ist, wobei der gegenüber liegend angeordnete Oberflächenbereich des hinteren Badkontakt-Bereiches frei von diesem Material ist, wird sichergestellt, dass der vordere Bereich des Badkontaktes besser vor der Metallschmelze (beispielsweise Stahlschmelze) geschützt ist als der hintere Bereich des Badkontaktes. Dadurch wird dieser hintere Bereich zuerst zerstört. Dabei bildet sich eine gegenüber dem Querschnitt des Messkopfes geneigte Flächenstruktur aus, wobei der vordere Badkontakt-Bereich am oberen, in Eintauchrichtung vorderen Niveau angeordnet ist und die Fläche zum Rand hin in zur Eintauchrichtung entgegengesetzte Richtung abfällt. Dadurch können Gasblasen, die sich beim Eintauchen an der Frontseite des Badkontaktes bilden, sich dort nicht sammeln, sondern sie werden seitlich nach hinten abgeleitet, so dass der Kontakt zwischen Stahlschmelze und Badkontakt nicht gestört ist.

Vorzugsweise erstreckt sich der Badkontakt in Eintauchrichtung vor der Außenfläche des Messkopfes. Die beiderseitige Anordnung des beständigen Materials erstreckt sich zweckmäßigerweise vom vorderen Badkontakt-Bereich bis zum Messkopf. Des weiteren ist es zweckmäßig, dass der hintere Badkontakt-Bereich und der vordere Badkontakt-Bereich in einer senkrecht zur Eintauchrichtung angeordneten Projektionsebene nebeneinander angeordnet sind. Der vordere Badkontakt-Bereich ist vorzugsweise in Eintauchrichtung mindestens 3 mm vor dem hinteren Badkontakt-Bereich angeordnet, um die Gase besser abzuleiten, da bei diesem Mindestabstand die Abschrägung / das Gefälle ausreichend groß ist. Der Badkontakt kann auch aus einem Blech gebildet sein. Er kann auch aus einem Metallstab, beispielsweise wie im vorstehend beschriebenen Stand der Technik genannt, gebildet sein, wobei dann das beständige Material asymetrisch um den Metallstab herum angeordnet ist. Dadurch werden auf diese Weise auch ein hinterer und ein vorderer Badkontakt-Bereich gebildet. Wichtig für den hinteren Badkontakt-Bereich ist es, dass der Badkontakt selbst dort nicht so gut vor der Metallschmelze (Eisen- oder Stahlschmelze) geschützt ist wie der vordere Badkontakt-Bereich, so dass er sich im hinteren Bereich schneller auflöst unter Bildung der beschriebenen hügeligen Struktur.

Vorteilhaft ist es weiterhin, dass der, insbesondere aus einem Blech gebildete, Badkontakt um eine etwa parallel zur Eintauchrichtung angeordnete Achse gebogen ist, wobei ein Teil des Bleches innerhalb der durch das Biegen gebildeten Silhouette angeordnet ist. Das beständige Material füllt vorzugsweise die Silhouette aus und lässt Teile des Badkontaktes am Außenumfang frei. Als beständiges Material wird vorzugsweise Gießereisand oder Zement verwendet.

Nachfolgend werden Ausführungsbeispiele der Erfindung an Hand von Zeichnungen erläutert. In den Zeichnungen zeigt:
- Fig. 1: den Messkopf eines Probennehmers mit Badkontakt,
- Fig. 2: einen Messkopf mit einer weiteren Ausführung eines Badkontaktes,
- Fig. 3: einen Messkopf mit einer weiteren Ausführung eines Badkontaktes und
- Fig. 4: verschiedene Badkontakte (Fig. 4a, 4b, 4c).

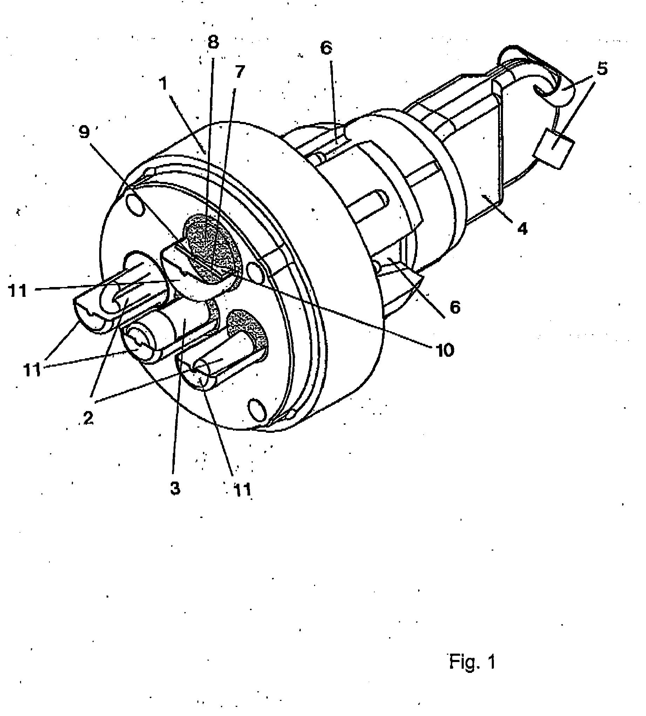

Figur 1 zeigt den Messkopf 1 eines Probennehmers. An seinem Eintauchende sind Sensoren 2 angeordnet sowie das Einlaufrohr 3 einer am rückseitigen Ende des Messkopfes 1 angeordneten Probenkammer 4. Die Probenkammer 4 ist eine übliche 2-schalige Probenkammer, die an ihrem Ende mit Klammem 5 zusammengehalten ist. Am rückseitigen Ende des Messkopfes 1 wird ein in der Zeichnung nicht dargestelltes Trägerrohr aufgesteckt. Erkennbar sind am Messkopf 1 Nischen 6 zur Kontaktierung der rückseitigen Anschlüsse der Sensoren 2. Zusätzlich ist an dem Eintauchende des Messkopfes 1 ein Badkontakt 7 angeordnet. Der Badkontakt 7 hat in dem Ausführungsbeispiel der Figur 1 die Form eines etwa ebenen Bleches. Er ist eingebettet in feuerfesten Zement 8, der in seinem vorderen Badkontaktbereich 9 (etwa in der Mitte) beidseitig vom Zement 8 bis zu seiner vorderen Stirnkante umgeben ist. Der hintere Badkontakt-Bereich 10 ist der Stahlschmelze beim Eintauchen relativ ungeschützt ausgesetzt, er wird abgeschmolzen. Die in der Figur 1 dargestellte kegelige Form des Zements 8 ermöglicht das Abführen von Gasen aus dem Bereich der Stirnseite des Badkontaktes 7, so dass eine einwandfreie Kontaktierung mit der Stahlschmelze möglich ist. Badkontakt 7, die Sensoren 2 und das Einlaufrohr 3 sind von Schutzkappen 11 umgeben.

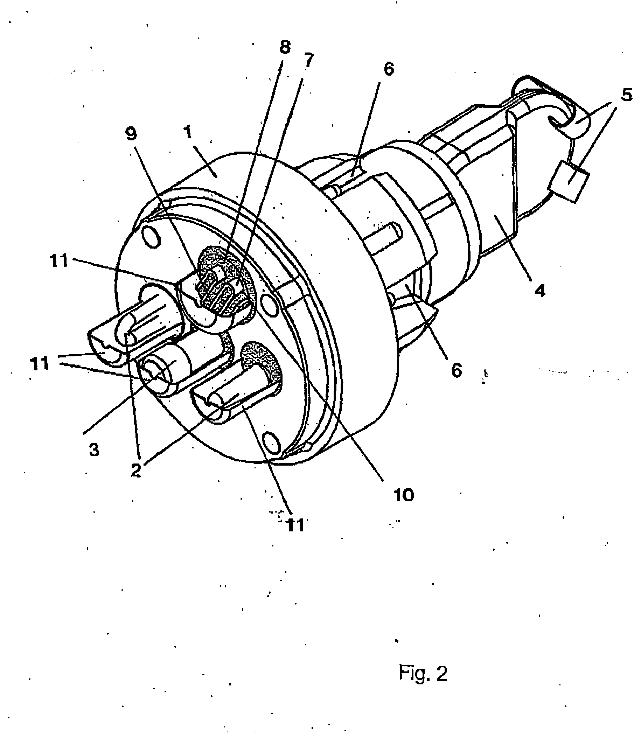

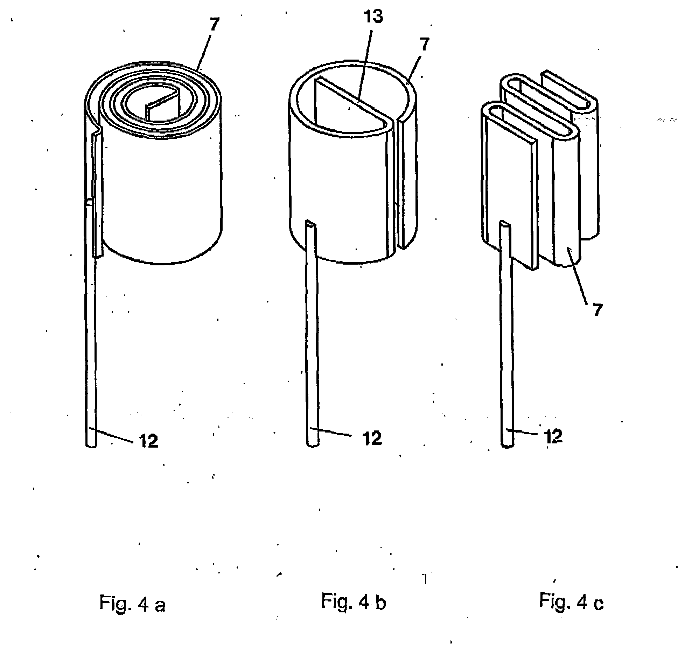

In Figur 2 ist ein ähnlicher Messkopf 1 dargestellt. Im Gegensatz zum Ausführungsbeispiel nach Figur 1 ist der in Figur 2 dargestellte Badkontakt 7 mäanderförmig ausgebildet mit einem vorderen Badkontakt-Bereich etwa im Zentrum des Badkontaktes 7 und einem hinteren Badkontakt-Bereich am äußeren Umfang. Ein solcher Badkontakt ist in Figur 4c dargestellt. Er wird durch eine Bohrung im Messkopf 1 hindurch mittels eines Kontaktes 12 in einer Nische 6 mit einer Kontaktleitung verbunden.

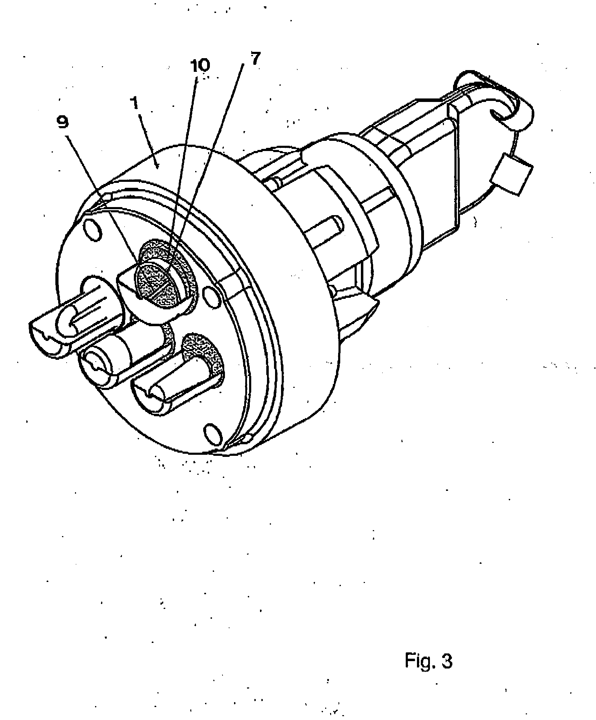

Figur 3 stellt eine weitere Ausführungsform eines Badkontaktes 7 dar bei im übrigen gleicher Ausbildung des Messkopfes 1. Der Badkontakt 7 ist als kreisförmig gebogenes Blech ausgebildet, dessen eines Ende 13 in den Kreis hinein abgebogen ist (vgl. Figur 4b). Die Kontaktierung erfolgt bei allen gezeigten Ausführungsbeispielen in gleicher Weise. Der vordere Badkontakt-Bereich 9 befindet sich etwa im Zentrum des durch den Badkontakt 7 gebildeten Kreises, während sich der hintere Badkontakt-Bereich 10 am äußeren Umfang befindet. Der Kreis ist im inneren vollständig oder nahezu vollständig mit Gießereisand oder Zement ausgefüllt.

Figur 4a zeigt eine weitere Ausführungsform des Badkontaktes 7. Hier ist im Gegensatz zu der in Figur 4b dargestellten Ausführungsform nicht nur eine ebene Fläche in das Kreisinnere abgebogen , sondem das Blech ist in Form einer Spiralfeder aufgewickelt.

## Patentansprüche

1. Messsonde zur Messung in Metallschmelzen mit einem an einem Eintauchende eines Trägerrohres angeordneten Messkopf, an dem mindestens ein Sensor zur Bestimmung einer Komponente der Metallschmelze und ein Badkontakt angeordnet sind, wobei der Badkontakt einen, in Eintauchrichtung gesehen, vorderen Badkontakt-Bereich aufweist mit jeweils zwei senkrecht zur Eintauchrichtung gegenüberliegend angeordneten Oberflächenbereichen, **dadurch gekennzeichnet, dass** an den gegenüberliegend angeordneten Oberflächenbereichen des vorderen Badkontakt-Bereichs ein in Eisen- oder Stahlschmelze beständiges Material angeordnet ist und dass an einem hinteren Badkontakt-Bereich lediglich an höchstens einem Oberflächenbereich ein in Eisen- oder Stahlschmelze beständiges Material angeordnet ist, wobei der gegenüberliegend angeordnete Oberflächenbereich des hinteren Badkontakt-Bereiches frei von diesem Material ist.

2. Messsonde nach Anspruch 1, **dadurch gekennzeichnet, dass** der Badkontakt sich in Eintauchrichtung vor der Außenfläche des Messkopfes erstreckt.

3. Messsonde nach Anspruch 1 oder 2, **dadurch gekennzeichnet, dass** die beidseitige Anordnung des beständigen Materials sich vom vorderen Badkontakt-Bereich bis zum Messkopf erstreckt.

4. Messsonde nach einem der Ansprüche 1 bis 3, **dadurch gekennzeichnet, dass** der hintere Badkontakt-Bereich und der vordere Badkontakt-Bereich in einer senkrecht zur Eintauchrichtung angeordneten Projektionsebene nebeneinander angeordnet sind.

5. Messsonde nach einem der Ansprüche 1 bis 4, **dadurch gekennzeichnet, dass** der vordere Badkontakt-Bereich in Eintauchrichtung mindestens 3 mm vor dem hinteren Badkontakt-Bereich angeordnet ist.

6. Messsonde nach einem der Ansprüche 1 bis 5, **dadurch gekennzeichnet, dass** der Badkontakt aus einem Blech gebildet ist.

7. Messsonde nach Anspruch 6, **dadurch gekennzeichnet, dass** der Badkontakt um eine etwa parallel zur Eintauchrichtung angeordnete Achse gebogen ist, wobei ein Teil des Bleches innerhalb der durch das Biegen gebildeten Silhouette angeordnet ist.

8. Messsonde nach Anspruch 7, **dadurch gekennzeichnet, dass** das beständige Material die Silhouette ausfüllt.

9. Messsonde nach einem der Ansprüche 1 bis 8, **dadurch gekennzeichnet, dass** das beständige Material Gießereisand oder Zement ist.
